(19) **Europäisches Patentamt / European Patent Office / Office européen des brevets**

(11) **EP 1 909 908 B1**

(12) **EUROPEAN PATENT SPECIFICATION**

(45) Date of publication and mention
of the grant of the patent:
**30.03.2011 Bulletin 2011/13**

(21) Application number: **06744068.5**

(22) Date of filing: **02.06.2006**

(51) Int Cl.:
*A61N 7/00* *(2006.01)*     *A61N 7/02* *(2006.01)*
*A61M 37/00* *(2006.01)*     *A61B 5/055* *(2006.01)*
*A61B 17/22* *(2006.01)*     *A61B 17/00* *(2006.01)*

(86) International application number:
**PCT/GB2006/002014**

(87) International publication number:
**WO 2006/129099 (07.12.2006 Gazette 2006/49)**

(54) **ULTRASOUND TREATMENT SYSTEM**

ULTRASCHALLBEHANDLUNGSSYSTEM

SYSTEME DE TRAITEMENT A ULTRASONS

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **02.06.2005 GB 0511259
02.06.2005 US 686411 P**

(43) Date of publication of application:
**16.04.2008 Bulletin 2008/16**

(73) Proprietor: **Cancercure Technology AS
0307 Oslo (NO)**

(72) Inventor: **MYHR, Gunnar
0307 Oslo (NO)**

(74) Representative: **Piésold, Alexander James et al
Dehns
St Bride's House
10 Salisbury Square
London
EC4Y 8JD (GB)**

(56) References cited:
**EP-A- 0 351 610**     **EP-A- 0 558 029**
**WO-A-2004/016311**     **US-A- 5 370 121**
**US-A- 5 490 840**     **US-A- 5 827 204**
**US-A- 5 984 882**     **US-A- 6 113 558**
**US-B1- 6 461 586**     **US-B1- 6 623 430**
**US-B1- 6 740 039**

**Description**

The present invention relates to an ultrasound treatment system. In certain aspects, it relates to the multi modal delivery and release of agents, such as a drug or gas, within a living creature, for example for the treatment of cancer or thrombi, under a non-invasive regime. In other aspects, the invention relates to an ultrasound system for inducing hyperthermia, ablation or causing tissue destruction, such as necrosis or apoptosis.

The present invention is a further development of the applicant's prior International Patent Application WO 02/15976 "Apparatus for selective cell and virus destruction within a living organism" and WO 05/002671 "Therapeutic Probe", which are hereby incorporated by reference.

Probably the most common method of releasing drugs in a controlled fashion utilizes coatings. Generally, with this method the drug is coated with e.g. polymers or inorganic materials that have varying resistance to breakdown by the body.

Liposomes are and will probably be the most successful carrier system for targeting the delivery of drugs. Liposomes are colloidal, vesicular structures based on lipid bilayers. Liposome encapsulated drugs are inaccessible to metabolising enzymes; prolong drug action, have directional potential, can act as non-vital transfection systems and can be used as adjuvants in vaccine or drug formulations.

A serious limitation of conventional chemotherapy is that cytotoxic drugs do not target cancer cells specifically, but affect essentially all tissues containing dividing cells. Such effects are exerted in various stages of the cell cycle. To get specificity and improve stability of chemotherapeutic drugs, lipid encapsulation has been introduced. Thus, the time of circulation of the drugs in blood can be increased, by protection of the drug molecules in the lipid particles, and, at the same time, avoiding general tissue penetration due to size considerations. Increased selectivity has been achieved by, for instance, inserting pH-responsive copolymers into the liposomal membranes, and exploiting the acidic environment of endosomes in cancer cells [Farmacological Rev. 51(4) (1999) 692 - 737]. Another means of achieving tumour selectivity is the use of enzyme activated prodrug therapy. This is a two step approach, where in the first step a drug-activating enzyme is targeted and expressed in the tumours. In the second step, a non-toxic prodrug, a substrate of the exogenous enzyme that is subsequently to be expressed in tumours, is administered systemically, in order to get high local concentration of the anticancer drug in the tumours [Clin. Cancer Res. 7 (2001) 3314 - 3324]. In clinical trials, liposomally encapsulated doxorubicin gives less cardiotoxicity but does not give any significantly improved progression-free survival for women with metastatic breast cancer, as compared to doxorubicin alone [Cancer 94(1) (2002) 25 -36, Annals of Oncology 15 (2004) 440 - 4493]. However, encapsulated doxorubicin in combination with paclitaxel, seems to be promising [Cancer Chemother. Pharmacol. 53(5) (2004) 452 - 457, Breast 13(3) (2004) 219 - 2266]. Antibody-directed enzyme prodrug therapy (ADEPT) is a targeted therapy in which a prodrug is activated selectively in the tumour by an enzyme, which is targeted to the tumour by an antibody (antibody-enzyme conjugate). ADEPT may offer some options for improved treatment [Br. J. Cancer 87 (6) (2002) 600 - 6007, Br. J. Cancer 9(12) (2004) 2402 - 24107].

A delivery system that released the drug from stabilized micelles into tumours by application of low-frequency US has been described [Cancer Res 62(24) (2002) 7280-7283]. Doxorubicin was encapsulated in stabilized Pluronic micelles and administered weekly intravenously for four weeks. The tumours were exposed to (primarily) 70 kHz US. Subsequent application of US reduced the tumour size. However, due to scatter in tumour growth patterns, none of the individual treatment group differences were statistically significant.

Myhr and Moan "Synergistic and tumour selective effects of chemotherapy and ultrasound treatment", [Cancer Letters 232 (2006) 206-213], have analysed the effects of low frequency ultrasound (20 kHz) exposure in combination with liposomally encapsulated doxorubicin (Caelyx) and Plurogel encapsulated fluorouracil (5-FU) on 144 Balb/c nude mice inoculated with a WiDr (human colon cancer) tumour cell line, at various concentrations. For the first time it was shown that non-hyperthermic ultrasound treatment significantly increases the effect of liposomally encapsulated cytostatic drugs on tumour growth. Synergetic effects were larger for low drug concentrations, indicating that the approach may benefit patients for whom chemotherapeutic treatment gives limited effect, or for whom drug concentrations have to be restricted due to general health considerations.

Blood clots (fibrin clots) are the clumps that result from coagulation of the blood. A blood clot that forms in a vessel or within the heart and remains there is called a thrombus. A thrombus that travels from the vessel or heart chamber where it formed to another location in the body is called an embolus, and the disorder, an embolism (for example, pulmonary embolism). Sometimes a piece of atherosclerotic plaque, small pieces of tumour, fat globules, air, amniotic fluid, or other materials can act in the same manner as an embolus. Thrombi and emboli can firmly attach to a blood vessel and partially or completely block the flow of blood in that vessel. This blockage deprives the tissues in that location of normal blood flow and oxygen. This is called ischemia and if not treated promptly, can result in damage or even death of the tissues (infarction and necrosis) in that area.

Deep venous thrombosis (DVT) refers to a blood clot embedded in one of the major deep veins of the lower legs, thighs, or pelvis. A clot blocks blood circulation through these veins, which carry blood from the lower body back to the heart. The blockage can cause pain, swelling, or warmth in the affected leg.

Blood clots in the veins can cause inflammation (irritation) called thrombophlebitis. The most worrisome complications of DVT occur when a clot breaks loose (or embolizes) and travels through the bloodstream and causes blockage of blood vessels (pulmonary arteries) in the lung. This can lead to severe difficulty in breathing and even death, depending on the degree of blockage.

In the United States, about 2 million people per year develop DVT. Most of them are aged 40 years or older. Statistics reveal that at least 200,000 patients die each year from blood clots in their lung.

The technology and procedures described herein are equally applicable to the treatment of blood clots as cancerous tissues.

The major problem with conventional cancer treatment options, if you can not remove all the cancerous tissues surgically, is general toxicity. This limits the amount of drug which can be administered to the patient. The challenge is to obtain significant local release of the drug(s) within a region of interest to destroy the tumour, while healthy tissues and the patient are unharmed.

Besides cytotoxic substances there are a variety of therapeutic drugs or cocktails of drugs that may be delivered to a cancerous area or volume within a patient (region of interest), and be acoustically released and/or provide enhancement to the therapeutic effects of the drugs. Among these are phototherapeutic substances, radiation sensitizers (together with ionizing radiation) and anti-angiogenetic agents. The actual release mechanism, ultrasound, may also be programmed to cause hyperthermia, with or without the use of parametric generation of low frequency ultrasound. However, the thermal energy will most likely be added separately by different frequencies and/or different transducers. In either way, targeted ultrasound can cause hyperthermia and as such contribute as an additional (and enhancing) treatment modus.

Photodynamic therapy (PDT) is a treatment that combines a photosensitizer with light to generate oxygen-dependent photochemical destruction of diseased tissue. This modality has been approved worldwide since 1993 for the treatment of several oncological and non-oncological disorders.

A tumour consists of two fundamental elements: parenchyma (neoplastic cells) and stroma. The stroma is composed of vasculature, cellular components, and intercellular matrix and is necessary for tumor growth. All the stromal components can be targeted by PDT. Evidence has indicated that effective PDT of tumour requires destruction of both parenchyma and stroma. Further, damage to subendothelial zone of vasculature, in addition to endothelium, also appears to be a crucial factor. [Ultrastruct Pathol. 2004 Sep-Dec; 28(5-6):333-40].

Some substances are of particular relevance related to the context of acting as adjuvants in relation to aminolevulinic acid (ALA) and/or ALA esters: US Patent No. 5,753,259 provides a method and product for preparing a controlled-release composition. US Patent No. 6,656,385 describes functionalized cubic gel precursors, functionalized cubic liquid crystalline gels, dispersions of functionalized cubic gel particles, functionalized cubic gel particles, and methods of preparation and use thereof. The precursors, gels, dispersions, and particles can be used to deliver active ingredients to substrates.

The problem of applying photochemotherapy subcutaneously, is that in its traditional administration it requires an invasive activating device, however photo dynamic substances can also be activated by the use of ultrasonic energy.

The common slow-growing solid tumours are resistant to most cytotoxic drugs. Among several factors influencing resistance is the degree of intra-tumoural hypoxia. The proportion of hypoxic cells in a tumour is, in part, a function of tumour size, but even small tumours (1 mm in diameter) may have hypoxic fractions ranging from 10-30%. The tumour types in which significant hypoxic fractions have been identified include all the common solid tumours, especially lung, colon, head and neck and breast cancers.

Hypoxia has been recognized to confer resistance to radiation therapy. Important gains in the efficacy of radiation have been achieved by a focus on diminishing radiobiological hypoxia, most recently with hyperfractionation. A randomized trail in locoregional lung cancer supports the improved therapeutic efficacy of this approach. Several findings were observed in cancers, substantially poorer response to therapy and survival were associated with median tumour $pO_2$ < 4mm Hg. Additional evidence supports a role for hypoxia in tumour progression to a more aggressive phenotype. Thus, tumour cell hypoxia is a significant barrier to effective cancer therapy, and its reversal is a therapeutic priority, [www.med.upenn.edu/pharm/faculty/indexy.html].

Chemotherapeutic agents that are highly responsive to ionizing radiation and enhance the effectiveness of radiation treatment are termed radiation sensitizers. Radiation sensitizers act in a number of ways to make cancer cells more susceptible to death by radiation than surrounding normal cells, and several such compounds are available for the treatment of solid tumours [Oncology (Dec 2003);17(12 Suppl 13):23-8]. The ideal radiation sensitizer would reach the tumour in adequate concentrations and act selectively in the tumour compared with normal tissue. It would have predictable pharmacokinetics for timing with radiation treatment and could be administered with every radiation treatment. The ideal radiation sensitizer would have minimal toxicity itself and minimal or manageable enhancement of radiation toxicity. Such a substance does not exist.

A concept of combining 2 modalities of cancer treatment, radiation and drug therapy, is described [Journal of Nuclear Medicine Vol. 46 No. 1 (Suppl) 187S-190S]; to provide enhanced tumour cell kill in the treatment of human malignancies and discusses molecules that target DNA and non-DNA targets. However, single modal cancer treatment based on radiation or chemotherapeutic substances, and some double modality treatment options with the additional use of radiation sensitizers, have shown limited success due to general toxicity concerns.

Standard procedure for e.g. breast cancer is adjuvant treatment based on surgery, chemotherapy and radiation exposure in sequence. Adjuvant treatment, in this context, is the prophylactic (protective) use of local (radiation) or systemic (cytostatic) treatment following primary procedure (surgery). In large randomized trials 10 years survivability for women with breast cancer (all stages) tend to increase by 6 % to approximately 57 %. In advance it is not possible to target the individuals who will benefit from the adjuvant treatment, thus it is standard procedure offered to most women.

There is clearly a need for a multimodal, selective and non-invasive cancer treatment option.

Angiogenesis is a word that comes from combining the two Greek words angio, meaning "blood vessel," and genesis, meaning "beginning." Angiogenesis is the creation of tiny new blood vessels. Normally, angiogenesis is a healthy process. New blood vessels develop, for instance, to help the body to heal cuts and other wounds. But during cancer, the same process creates new, very small blood vessels that provide a tumour with its own blood supply. Anti-angiogenesis treatment is the use of drugs, other substances or biophysical procedures to stop tumours from developing new blood vessels. Without a blood supply, tumours cannot grow beyond initial cell divisions [Current Cancer Drug Targets (November 2004) vol. 4, no. 7, pp. 555-567].

Vascular targeting agents (VTAs) for the treatment of cancer are designed to cause a rapid and selective shutdown of the blood vessels of tumors. Unlike anti-angiogenic drugs that inhibit the formation of new vessels, VTAs occlude the pre-existing blood vessels of tumors to cause tumour cell death from ischemia and extensive hemorrhagic necrosis. Ligand-based VTAs use antibodies, peptides, or growth factors that bind selectively to tumour versus normal vessels to target tumors with agents that occlude blood vessels. The ligand-based VTAs include fusion proteins (e.g., vascular endothelial growth factor linked to the plant toxin gelonin), immunotoxins (e.g., monoclonal antibodies to endoglin conjugated to ricin A), antibodies linked to cytokines, liposomally encapsulated drugs, and gene therapy approaches. [Clin Cancer Res. (Jan 2004);10(2):415-27].

Hyperthermia is a therapy technique by means of increasing the temperature of cancer tissue several degrees above normal body temperature (41 to 45 °C), thus enhancing the therapeutic effect, of conventional therapies. Thermal destruction, such as necrosis or apoptosis, is a means of heating the tissue in question to 60°C - 80 °C, thus causing permanent damage. Radio resistance (the immunity to the radiation) of over-exposed patients to radiotherapy tends to increase. By increasing the cancer tissue temperature using hyperthermia, its radio resistance will decrease and, therefore, the therapy combination of hyperthermia and radiotherapy will improve the quality of therapy. Furthermore, hyperthermia can increase the effectiveness of chemotherapy because the said technique also has positive impact to chemical reaction kinetics.[Int J Hyperthermia (Nov 2004);20(7):781-802].

It is known from US Patent No. 5275165 to use a focused ultrasound transducer selectively to destroy tissue in a region within a subject. The transducer, having a fixed focal length, is positioned in an ultrasound conducting liquid below a table on which the patient lies and is arranged to be moved in the "X", "Y" and vertical directions so as to focus on different locations within the patient. The energy produced by the ultrasound transducer is focused onto a tumour and pulsed to selectively heat the tumour. An operator is provided with cross-sectional temperature sensitive images by the use of magnetic resonance imaging apparatus and the transducer positioning means is responsive to a manually operated control unit. Similar systems are disclosed in US Patents Nos. 5443068 and 5769790.

Microbubbles occur naturally within fluids, and consequently within living creatures. On a micro level, finite vapour pockets are formed due to molecular movements and vacancies. Macroscopically, saturated vapour, gas and liquid are balanced within a fluid related to pressure and temperature. Also, fluids contain solid particles and micro bubbles of contaminant gas and air. In addition, thermal motions within a liquid can form temporary, microscopic voids that can constitute the nuclei necessary for rupture (of pockets and microbubbles) and cause growth to macroscopic bubbles, [Brennen, C.S. Cavitation and bubble dynamics, Oxford University Press, (1995)].

Cavitation involves the nucleation, growth and oscillation of gaseous and/or vapour cavities within a fluid, stabilized on solid surfaces or by surface active films, owing to the reduction of pressure in the negative part of the acoustic cycle. The phenomena represent either rapid growth and collapse of bubbles, called inertial or transient cavitation, or cause sustained oscillatory motion of bubbles, named stable cavitation.

Stable oscillations or acoustic streaming of bubbles induce fluid velocities, vortices and exert shear stress on surrounding cells and tissues, while transient cavitation causes rapid isotherm growth and collapse of bubbles. The collapse of bubbles can be sudden (microseconds) and adiabatic, causing momentary high temperatures (T > 5000 K) in the bubble core, light (sonoluminance) and the formation of shock waves (p > 800 atom), capable of disrupting tissues and enhancing drug transport across membranes. Such high temperatures can cause free-radical formation which might damage surrounding biological matter in much the same way that ionizing radiation does. Collapsing bubbles near a surface or boundary experience non-uniformities in their surroundings that result in the formation of high velocity micro jets. The micro jet can penetrate tissues and/or capsulations (liposomes, polymers) causing secondary stress waves, [Ultrasound in Med. & Biol. (1991) 17; 179-185], [Nature Reviews (2005) 80; 255-260].

Microbubbles, currently used as contrast agents, have potential therapeutic applications. Microbubbles, upon insonation of sufficiently intense ultrasound will cavitate. Cavitation of microbubbles naturally occurring and/or added as a cocktail together with drugs (coadministration) and/or in an encapsulated form (e.g. within liposomes or polymer coatings), can be used to dissolve blood clots or deliver drugs. Targeting ligands and drugs can be incorporated into microbubbles to make highly specific diagnostic and therapeutic agents for activation with selectively delivered ultrasound. This is discussed in Investigative Radiology, Vol. 33, No. 12, 886 - 892 and European Journal of Radiology 42 (2002) 160-168. The paper in Investigative Radiology proposes that ultrasound imaging could be used to localise a treatment volume, but there is no proposal for how this would be achieved.

In Myhr and Moan mentioned above and Nelson et. al. [Cancer Res 62(24) (2002) 7280-7283] and in references therein, it is concluded that both the release of encapsulated drugs and accompanying synergistic effects are more profound at lower ultrasonic frequencies. Myhr and Moan used 20 kHz and Nelson et. al. applied primarily 70 kHz. To induce the maximum release and/or uptake within a region of interest of drugs, encapsulated drugs within micelles, with or without added microbubbles, appropriate cavitational energy and/or frequency levels are pursued. An apparatus according to the preamble of claim 1 is known from US-B1-6461586. Cavitation is often referred to as dependent on the mechanical index, MI.

$$MI = (P_{neg})/(f^{1/2}), \text{ where } P_{neg} = \text{maximum negative pressure (in MPa) and } f = \text{frequency (in MHz).}$$

The cavitational activity is inversely related to frequency.

As an average the speed of sound in the human body is 1540 ms$^{-1}$. With f = 20 kHz the wavelength is 7.7 cm. At f = 250 kHz the wavelength is 0.6 cm. The problems with low ultrasonic frequencies are the lack of directivity and to restrict exposure for relatively small regions of interest These problems can be solved by applying a focused transducer or transmitter, e.g. parabolically shaped or as a phased array arrangement.

Energy absorption, attenuation, is a function of frequency. $I(r) = I_0 \exp[-\mu(f)r]$ where $I(r)$ = intensity at tissue depth r, $I_0$ = output intensity and $\mu(f)$ = intensity-absorption coefficient, which is a function of frequency.

Energy absorption increases with increasing frequency.

The challenge is to reach a well defined volume within the patient (i.e. a region of interest) with high intensity acoustic energy at a low frequency, enabling to limit the exposure to a relatively small region of interest, and at the same time minimizing the acoustic exposure to surrounding tissues.

The present inventor has recognised that existing technology available for targeting ultrasound for tissue heating or destruction can be adapted and integrated into an inventive system for a different application, namely to target ultrasound at energy and/or frequency levels sufficient to cause cavitation. This cavitation may increase the interaction between the applied ultrasound and drugs and/or encapsulated therapeutic agents.

The present inventor has also recognised that real time monitoring of the cavitational effects of ultrasound can be used to monitor the effectiveness of the treatment as it is carried out. In particular, the inventor has recognised that the real time monitoring of cavitation can be used to calculate the quantity of drug uptake in the region of interest and to spatially map that uptake within the region of interest.

According to a first aspect of the invention, there is provided apparatus for non-invasive patient treatment using ultrasound and a therapeutic agent, comprising an ultrasonic transmitting device for directing ultrasonic energy at a region of interest of a patient, monitoring means for real time monitoring of cavitation in and around the region of interest, and control means for receiving information from the monitoring means and controlling the ultrasonic transmitting device based on that information so that the ultrasonic energy is focused at the region of interest.

According to a second aspect of the invention, there is provided a method of non-invasive patient treatment using ultrasound and a therapeutic agent, comprising transmitting ultrasound and directing the ultrasonic energy at a region of interest of a patient, monitoring cavitation in and around the region of interest in real time, and controlling the ultrasonic transmitting device, based on the monitored cavitation, so that the ultrasonic energy is focused at the region of interest.

In this specification , the therapeutic agent may be a drug or it may be a gas. In the case of a gas, the collapse of the encapsulation releases energy which is used to provide a treatment e.g. to have a therapeutic effect on a blood clot. In the case of a drug, this may be encapsulated in the interior of capsules or attached to or incorporated in the membranes forming the capsule walls. Alternatively, a non-encapsulated drug may be used, administered for example orally or by injection. In that case, cavitation of naturally occurring microbubbles or microbubbles created by the ultrasound, takes place. The interaction of the ultrasound with the microbubbles may enhance the effects of the drug. In particular, the therapeutic agent may be a non-encapsulated drug, an encapsulated drug, an encapsulated drug containing gas bubbles or encapsulated bubbles with no drug. Mixtures of these therapeutic agents may also be used.

An appropriate diagnostic tool is preferably used to determine the region of interest with respect to a reference point in space, such as with respect to a fixed table or frame, or with respect to a reference point on the patient. This determination may take the form of a set of coordinates, e.g. in the "X", "Y" and "Z" directions. In a simple case, a single point in the patient may be determined as the desired location, or a three dimensional shape of one or more e.g. tumours or thrombi may be mapped.

The region of interest (tumour, thrombus, organ or the like) can be modeled by topographic modeling techniques (morphometry, digital elevation models, tumour profiling etc.). An adequate 3D or 3D + time digital (or analog) tumour model may facilitate the optimal automated or manually controlled treatment regime or procedure.

In preferred embodiments, using a digital diagnostic imaging device like Computer Tomograph, Magnetic Resonance Imaging, Positron Emission Tomograph and the like, stereometric coordinates to one or several regions of interest (tumours or blood clots) are established. The coordinates may be linked to one or several fiducial or reference points within or on the body. Such reference points may be the top of the ear, the tip of the nose, a certain location on the skeleton and/or a dye or a tattoo mark on the skin. The stereometric coordinates of the regions of interest and/or the (digital) tumour model may be recorded or computed by the control means, which is preferably a processing unit, with accompanying software to perform such tasks.

The diagnostic unit may be integrated with the apparatus, so for example there may be a diagnostic transmitter in addition to the therapeutic ultrasonic transmitting device. Alternatively, or additionally, the diagnostic unit may be provided separately. This may be useful where an existing diagnostic unit is already available. Likewise, an (ultrasonic) image transducer or device can be integrated within the therapeutic ultrasonic device for real time monitoring.

In a preferred embodiment, the apparatus of the invention is provided in combination with a diagnostic unit for determining the region of interest. The diagnostic unit may provide inputs for digital or analog (tumour) modeling. In one aspect, the present invention is a system comprising the diagnostic unit, the ultrasonic transmitting device, the monitoring means and the control means.

The diagnostic unit may be provided at a first station and the ultrasonic transmitting device may be provided at a second station. The patient will then need to be moved from the first station to the second station. In that case, it is beneficial if the reference point relative to which the desired location for treatment is determined is on the patient or on a table or frame relative to which the patient does not move between diagnosis at the first station and treatment at the second station.

Enhanced ultrasound contrast due to a small gas bubble has been observed for almost as long as medical ultrasound equipment has been in clinical use.

The drugs, the (e.g. liposomally) encapsulated drugs, with or without microbubbles, cocktails of drugs and/or microbubbles, are systemically or locally administered. During the therapeutic session, microbubbles, located within the region of interest, will explode and release the encapsulated drug or drugs and/or enhance the effects of the drug(s).

The collapse of micelles, liposomes or other encapsulations (e.g. polymers) due to exploding microbubbles and the release of the drug or drugs (cocktail) will be an inverse function of the contrast. The diminishing contrast within a region of interest can be visually displayed and digitally or analogously recorded or mapped. The inventor has found that cavitational effects in general can be monitored by diagnostic ultrasound units as well as by MRI. These findings provide novel means for controlling an ultrasound therapeutic treatment system.

The apparatus therefore comprises monitoring means for monitoring the cavitational effects and/or the release of a therapeutic agent at the region of interest in real time. Sufficient, but not excessive, energy levels and/or frequencies to facilitate such cavitational effects are provided by transmitting means discussed elsewhere in this document.

The information provided by the monitoring means may relate to the amount of agent released and/or it may relate to the location where the agent is released. In the former case, the monitoring means may provide information concerning the agent dosage delivered and/or released at the region of interest, as a result of the interaction of the agent with the ultrasonic energy. The system can therefore terminate the therapeutic session, endogenously or exogenously, based on actual real time recording, preset, time (duration) or empirically achieved target values. The monitoring capability can also be used to control the administration of the agent to the patient based on the information concerning the agent dosage delivered. For example if the drug or the agent is being administered intravenously during the ultrasound treatment, the administration of the drug or the agent can be terminated or modified in response to information provided by the monitoring process.

In the case of the monitoring means providing information relating to the location where the agent is released, the coordinates of the volume within the patient where the agent is actually being released may be determined and then compared to the previously determined coordinates of the region of interest. If there is a difference (beyond certain tolerance limits), then the control means can control the ultrasonic transmitting device so that the focus of the ultrasound energy is adjusted taking account of the difference, to ensure that the ultrasound energy is correctly focused. In effect, a feedback is provided in real time to improve the accuracy of agent delivery. Alternatively or additionally to an automatic feedback to the control means, the apparatus may provide a display of the difference and/or a warning to the system operator.

Monitoring and/or measurement of cavitation or cavitational activities, volume or location of release, related to drug intake or administration, may be bridged to the various control functions by one or more data processing units, which may include appropriate software to conduct analysis of the input data, compare such results with specifications and provide output data which may conduct control functions.

As discussed above, the cavitation of microbubbles (either natural or added as part of an administered therapeutic agent, with or without encapsulated drugs) alters the permeability of the cell membrane to any drug which is present. This effect is known as sonoporation. The relationship between the level of cavitation and the rate of drug uptake by tissue in the region of interest (both target tissue and non-target tissue) can be found. Therefore, by monitoring the level of cavitation in real time, the level of drug uptake by the tissue in the region of interest can be calculated in real time and the overall effect of the treatment can be monitored. For example, if it is judged (manually by an operator or automatically by a control unit) that too much drug is being taken up by non-target tissue, the focus of the therapeutic ultrasound transmitter can be adjusted to focus more accurately on the target tissue or the treatment can be stopped. Also, if the treatment requires a certain quantity of drug uptake by the target tissue in the region of interest, that level can be accurately monitored and the duration of the treatment can be accurately controlled.

By spatially monitoring the level of cavitation in the region of interest and adjusting in real time the focus of the therapeutic ultrasound transmitter, the treatment can be controlled so as to minimise damage to non-target tissue while still maintaining an adequate level of therapeutic treatment to the target tissue.

When the real time monitoring of the cavitation in the region of interest and the empirically found relationship between the cavitation and the drug uptake is combined with the real time monitoring of the movement of the region of interest due to the patient's breathing and heartbeat (for example), and the focus of the therapeutic ultrasound transmitter is controlled accordingly so as to minimise damage to non-target tissue, a very precise treatment can be carried out even on critically located tumours near vital organs where damage to the surrounding tissue could have serious consequences, such as in the brain.

In a preferred embodiment of the invention, the monitoring of cavitation is carried out by a magnetic resonance imaging (MRI) machine.

In some circumstances there will be a temperature increase associated with drug or agent release, normally, but not limited to, less than 4°C in non-hyperthermic conditions. This can provide a way of providing feedback regarding the location where agent release is occurring in addition to monitoring cavitational effects. A thermal detection device may be provided for real time thermal monitoring e.g. recording, mapping and/or the establishment of the coordinates of a heated volume based on input from the thermal detection device (based e.g. on a temperature gradient in the X, Y and Z directions) within the patient. These coordinates may be compared to the region of interest and the result used by the control means as described above.

There may be thermal monitoring of a wider volume than only the region of interest. If there is a temperature increase elsewhere in the patient, the control means can adjust the ultrasound transmitting device and/or provide a display or warning to the operator.

Control of the ultrasonic transmitting device may be substantially automatic, or there may be additional manual operator input. In the case of automatic control, the information about the region of interest (e.g. the 3D tumour model) where the ultrasound energy is to be focused combined with the information from the monitoring means will be sufficient to carry out a predetermined treatment programme. For example, for a relatively small tumour or thrombus, a single focal point may be used so that the ultrasound energy causes cavitation and potentially interacts with any drug and/or encapsulated agent at that point. Alternatively, if a larger region of interest is mapped and/or a digital tumour model is developed, regardless of the tumour size, the control means may cause a series of ultrasound transmissions at different points throughout the region, following a predetermined pattern and/or an optimal treatment procedure. The control unit, with or without accompanying software, preferably has also the capability of optimizing the position of the therapeutic unit with respect to minimizing attenuation due to energy losses caused by bone, certain organs (e.g. lungs), natural cavities within the patient, and the like. The means of optimizing the position can be based on empirical values in relation to position data, and/or input from diagnostic and/or therapeutic devices.

In the case of control with additional operator input, an image of the region of interest may be provided on a screen and the operator can use the image to select points at which ultrasound energy is to be focused, using an input device such as a keyboard, mouse or the like. When a point has been selected by the operator, the control means controls the ultrasound transmitting device so that the ultrasound energy is focused at that desired location. The actual transmission may then be initiated by the operator.

The control means may have the capability of compensating for motion of the location where the ultrasound is to be focused caused by e.g. breathing, as described in International Patent Application WO 2004/075987 in relation to an ultrasound tissue destruction method. For example, a thrombus in or near the heart may move during treatment due to breathing and heart movements. Accordingly, the coordinates in the X, Y and Z directions and/or the digital morphometric model may be supplemented by an additional time dependent coordinate.

More than one ultrasonic transmitting device may be provided. Thus, one or several therapeutic transducers or transmitters may be orientated in a (preferably moveable) stereotactic configuration, each with an optimal or suboptimal energy intensity to minimize release of the encapsulated agent in tissue between the therapeutic transmitter and the region of interest. Where plural devices are provided, the emitted beams are preferably arranged about a circular arc and directed generally inwardly towards the therapeutic target. Thus the beams may be centred on the target. The transducers are preferably moveable and individually controlled.

Ultrasound beams may be focused by curving the piezoelectric plate (transmitter or transducer) or by interposing a lens or reflector between a flat plate and the target (region of interest). A phased array of transducers may be focused electronically. Also, focusing of the ultrasonic energy may be achieved by the geometric configuration of plural ultrasound transmitters, The intention is to minimise exposure to ultrasound of tissues outside the region of interest.

The phased array techniques represent the steering of the ultrasonic beam by means of electronic applied delays on the segments of a probe array. These delays are applied during emission (and reception) of the ultrasonic signals. To generate a beam, the various probe elements are pulsed at slightly different times. By precisely controlling the delays between the probe elements, beams of various angles, focal distance, and focal spot size can be produced. It is possible to change the angle, focal distance, or focal spot size, simply by changing the timing to the various elements.

The capacity to produce at will, and under computer control, various beam angles and focal lengths can be used to treat regions of interest (tumour models) with complex shapes under an automated optimal treatment regime or procedure.

Another possibility is the capability to generate a beam with a few probe elements and then to time-multiplex the beam to other elements of the probe. This, in effect, moves the beam along the probe axis, with no mechanical movement from the probe.

Also, by using circular array probes or curved (parabolic) transducers/transmitters, an ultrasound beam can be formed using a few probe elements and the beam can then be moved in a circular fashion by shifting the active probe element. [www.NDT.net - May 2002, Vol. 7 No. 05, www.NDT.net - Oct. 2000, Vol. 5 No. 10]

The present system may represent an overall control framework (which may include software algorithms) comprising a therapeutic ultrasound component and novel and inventive monitoring and control means for ultrasound mediated selective or targeted release of drugs or other therapeutic agents which may comprise and/or integrate existing technology available for targeting ultrasound for tissue heating or destruction.

In preferred arrangements the apparatus comprises a support for the ultrasonic transmitting device which is movable to adjust the position of the device relative to a patient. Such a support may take the form of a robotic arm. This type of construction allows for several possible positions of the ultrasonic transmitting device for a given desired location, allowing the apparatus to be adjusted if necessary to avoid the ultrasound having to be transmitted through problematic zones such as the lungs or bone. Also, at low frequencies standing waves may be a problem. This can be corrected by the movement of the ultrasonic transmitting device by, say, one half wavelength.

The ultrasound transmitting device may thus be fixed on a robotic arm, or on a circular arrangement, digitally controlled, located and guided by the control means. Several transmitters/transducers can transmit continuously or the transmissions can be pulsed. The transmitters can produce crossing acoustic beams, or the transmitters can emit in sequence, one or more at a time. A separate diagnostic transducer (also preferably fixed on a movable support such as a robotic arm), may be controlled and guided by the same control means e.g. processing unit.

In a preferred embodiment, electrical focusing of the ultrasound transmission is combined with mechanical motion of the ultrasound transmitting device. The concept of combining electrical focusing and mechanical motion has the advantages of both enlarging the acoustic window and providing dynamic focusing ability.

A system designed to provide hyperthermia has been described in Phys. Med. Biol. 48, 2003, 167-182. It facilitates conformal acoustic exposure (or heating) to a defined region of interest by the use of an external ultrasound source, by using a phased array transducer with mechanical motion. In this system, a one-dimensional phased array is arranged on a shaft and moves along the shaft, while dynamically focusing on the region of interest with numerous focal spots. To prevent overexposure/release (overheating) in the intervening tissue(s) between the skin and the region of interest, or elsewhere, the shaft and the phased array are rotated together to enlarge the acoustical window. With the purpose of conformal exposure/release (heating), the power deposition of the region of interest is constructed by combinations of the focal spots, and an iterative gradient descent method is then used to determine an optimal set of power weightings for the focal spots.

The feasibility of transcranial ultrasound focusing with a non-moving phased array and without skull-specific aberration correction was investigated using computer simulations, [Phys. Med. Biol. 50,2005, 1821-1836]. It was concluded that it is possible to focus a low-frequency (250 kHz) beam through skull without skull-specific aberration correction.

The patient may be positioned on a flexible membrane in contact with an ultrasound conducting medium in which the ultrasonic transmitting device is immersed, or a bag containing such a medium may be placed on the patient, with the ultrasonic transmitting device in contact with or located within the bag. Preferably, there is provided a vessel containing an ultrasound conducting medium and in which the ultrasonic transmitting device is disposed, the vessel being arranged to permit immersion in the ultrasound conducting medium of at least part of a patient's anatomy. Such an arrangement avoids a number of interfaces, such as gel to membrane, membrane to gel and gel to patient, and thereby eliminates the risk of air spaces. In this way a very good transmission of ultrasound energy can be obtained.

Thus, in preferred embodiments, between the ultrasonic transmitting device and the patient, there is water or gel to enhance acoustic coupling. The vessel may take the form of a bath in which the patient is at least partly immersed, possibly up to the neck. This allows positioning of the ultrasonic transmitting device (or devices) in a way to focus the ultrasonic energy as desired whilst avoiding regions of bone or the lungs between the transmitting device and the region of interest. The preferred arrangement in which the ultrasound transmitting device is provided on a movable support such as e.g. a robotic arm is particularly useful when the patient is at least partly immersed in an ultrasound conducting medium, because this allows considerable scope for different positions of the ultrasound transmitting device.

Ultrasound frequencies of less than 1 MHz are preferably used, more preferably less than 500 kHz. Therapeutic frequencies will typically be in the 20 kHz to 250 kHz range. Diagnostic frequencies will most often be in the 1.7 MHz to 3.4 MHz range, while hyperthermia, based on an independent treatment mode, is typically conducted in the 1 MHz to 3.4 MHz range.

The therapeutic ultrasound may be transmitted under non-hyperthermic conditions, i.e. without significantly heating the tissue, dependent on continuous or pulsed wave, frequency and/or intensity levels. Non-hyperthermic conditions are considered to be, but not limited to being, those when the temperature of the tissue is not increased by more than 4°C , i.e. not above about 41 °C in a human patient. Preferably the energy level of the ultrasound is sufficiently low that the ultrasound itself (i.e. without the use of encapsulated agents) would not cause tissue damage.

There may be provided one or more sensors to check the position of the region of interest during treatment. The sensor can provide a continuous update to the control means of the coordinates of the region of interest, allowing the system to update the position of the location where the ultrasound is focused. This will facilitate continued accurate agent and/or drug release if the patient is moved or if there are body movements during treatment. The sensor may be at least one active, reflective or passive sensor among e.g. laser, radio, electronic, heat, sound, infrared sensors and the like, positioned on, in or near the patient.

The coordinates/volume or model of the region of interest and the coordinates/volume of the agent release or heated volume can be mapped or displayed simultaneously.

As a quality assurance means, the control means may contain an algorithm which calculates the deposited energy within the region of interest, based on output intensity, emitted frequency, types and distances of penetration of various tissues. The result may be displayed to the system operator. If information provided to the control means in real time (such as a detected temperature increase) differs from what is expected based on the calculation (beyond certain tolerance limits), a warning may be provided to the system operator.

Several of the concepts, ideas or combinations of such which are discussed above, are considered to be of patentable significance in relation to ultrasound treatment with or without the release of encapsulated agents and/or in combination with existing devices or components which may be subjected to superior control and guidance.

According to another aspect, therefore, the invention provides a method of patient treatment using ultrasound, comprising determining the position of a region of interest in terms of spatial coordinates, storing or calculating an expected effect of therapeutic ultrasound on the region of interest, focusing ultrasound energy on the region of interest based on the spatial coordinates, measuring the effect of the ultrasound energy on the region of interest, comparing the measured effect with the stored or calculated effect, and providing an output of the comparison.

The invention also provides apparatus for patient treatment using ultrasound, comprising a diagnostic unit for determining the position of a region of interest in terms of spatial coordinates or morphometric model(s), a processing unit for storing or calculating an expected effect of therapeutic ultrasound on the region of interest, an ultrasonic transmitting device for focusing ultrasound energy on the region of interest based on the spatial coordinates or model(s), and a control, guidance and measuring device for measuring the effect(s) of the ultrasound energy on the region of interest and drug release and providing an output to the processing unit, the processing unit being arranged to compare the measured effect with the stored or calculated effect and provide an output of the comparison.

The output may then be used by an operator and/or internally by the apparatus better to achieve the desired treatment, for example by adjusting the focus of the ultrasound and/or its intensity or frequency. There will generally be certain tolerances set in the system which the comparison has to exceed before any adjustment is made.

The system may be used for non invasive ultrasound induced hyperthermia (heating to 41 - 45 degrees C) and/or ultrasound caused thermal destruction (causing necrosis by heating above 56 degrees, usually up to 60 - 80 degrees C) and/or ultrasound administration of a drug and/or an encapsulated agent. Thus the effect stored or calculated and then measured may be the change in temperature and/or the amount of drug or agent released.

The stored or calculated effect(s) may be based on known parameters such as tissue properties (e.g. density), the ultrasound intensity, or the ultrasound frequency.

The diagnostic unit may be arranged to determine the position of the region of interest dependent on time. So, the spatial coordinates may be supplemented by a time coordinate. This can take account of body movements of the patient and ensure that the ultrasound energy is correctly focused.

The various preferred features and options discussed in this specification in relation to treatment with a drug or an encapsulated therapeutic agent are also applicable to the above aspects of the invention, which, as mentioned may not involve the administration of an agent. Ionizing radiation or such capacity can be added or be an integral part of the system.

In one aspect, the invention relates to a method, apparatus and system for diagnosis, positioning, treatment and real time monitoring of drug and/or agent release, wherein (an) acoustic transducer(s) is (are) arranged in various orientations outside or within the body of a human or animal, herein denoted a patient. The acoustics interact with drugs or combinations of (various encapsulated) drugs and/or microbubbles for selective release within a region of interest or a modeled targeted volume.

The starting points of the therapeutic procedure are the diagnostic and location {coordinates [3D or 4D (3D in real time)]} of tumour(s), blood clot(s) or other region(s) of interest with respect to reference point(s)].

Most diagnostic radiographic systems in clinical use (such as chest and mammographic i.e. breast imaging) are based on the use of a phosphor screen. The phosphor screen emits light in response to x-ray absorption. The resulting optical image is conventionally used to expose a photographic film. Bone absorbs x-rays well and thus attenuates the beam. In this way the areas falling in the shadow of the bone appear light or underexposed on an x-ray film image because relatively few x-rays exit the patient and little light is produced in the phosphor screen. Traditional x-ray diagnosis represents an analog approach.

The word "tomography" is derived from the Greek words tomos (slice) and graphia (describing).

Computed axial tomography (CAT), computer-assisted tomography, computed tomography, CT, or body section roentgenography is the process of using digital processing to generate a three-dimensional image of the internals of an object from a large series of two-dimensional x-ray images taken around a single axis of rotation. The x-ray slice data is generated using an x-ray source that rotates around the object. X-ray sensors are positioned on the opposite side of the circle from the x-ray source. Many data scans are progressively taken as the object is gradually passed through the gantry.

In conventional CT machines, a vacuum tube containing a metal target onto which a beam of electrons is directed at high energy for the generation of x-rays. The x-ray tube is physically rotated behind a circular shroud.

In electron beam tomography (EBT) the tube is far larger, with a hollow cross-section and only the electron current is rotated.

The data stream representing the varying radiographic intensity sensed reaching the detectors on the opposite side of the circle during each sweep, 360 degree in conventional machines, 220 degree in EBT, are then computer processed to calculate cross-sectional estimations of the radiographic density.

CT is used in medicine as a diagnostic tool and as a guide for interventional procedures. Using contrast material can also help to obtain functional information about tissues.

The two forms of emission tomography are PET (Positron Emission Tomography or Positron Emitting Tracers) and SPECT (Single Photon Emission Computed Tomography). While SPECT is less expensive than PET, PET generally has better resolution, though multidetector camera's have raised SPECT's imaging qualities. PET and SPECT are selective and sensitive means for studying molecular pathways and molecular interactions in humans. While different tracers are used for each method, each method uses different tracers to highlight different aspects of the body.

Positron Emission Tomography (PET) gives physicians information about the chemistry of the body. Unlike CT or MRI, which look at anatomy or body form, PET studies metabolic activity and body function. PET has been used primarily in cardiology, neurology, and oncology. In particular, it has been used to assess the benefit of coronary artery bypass surgery, identify causes of childhood seizures and adult dementia, and detect and grade tumours. PET can determine flow rate and flow reserve in addition to metabolic activity.

PET is a branch of medicine that uses radioactive materials either to image a patient's body or to destroy diseased cells. One of two or more atoms with the same atomic number but with different numbers of neutrons (isotope), which decays by emitting a positron, are chemically combined with a metabolically active molecule, and is injected into the living subject (usually into the blood circulation). There is a waiting period while the metabolically active molecule (usually a sugar) becomes concentrated in tissues of interest, then the subject is placed in the imaging scanner. The short-lived isotope decays, emitting a positron. After traveling less than one millimeter the positron annihilates with an electron. Electromagnetic radiation are emitted during the radioactive decay and have an extremely short wavelength gamma ray photons moving in opposite directions. These are detected when they reach a scintillator material in the scanning device, creating a burst of light which is detected by photomultiplier tubes. The technique depends on simultaneous or coincidental detection of the pair of gamma photons. By measuring where the gamma rays end up, their origin in the body can be plotted, allowing the chemical uptake or activity of certain part of the body to be determined. The scanner uses the pair-detection events to map the density of the isotope in the body, in the form of slice images separated by about 5mm. The resulting map shows the tissues in which the molecular probe has become concentrated, and is read by a nuclear medicine physician or radiologist, to interpret the result in terms of the patient's diagnosis and treatment. PET scans are increasingly read alongside CT scans, the combination giving both anatomical and metabolic information (what the structure is, and what it's doing). PET is used heavily in clinical oncology.

Magnetic resonance imaging (MRI) was developed as an offshoot of nuclear magnetic resonance. The structural unit of an element is aligned in a powerful magnetic field. Then, radio frequency pulses are applied in a plane perpendicular to the magnetic field lines so as to cause some of the hydrogen nuclei to gradually change alignment from their upright positions. Magnetic field gradients are then applied in the 3 dimensional planes to allow encoding of the position of the atoms. After this, the radio frequency is turned off and the nuclei go back to their original configuration, but before doing so, their new alignment can be measured by coils wrapped around the patient. These signals are recorded and the resulting data are processed digitally. In clinical practice, MRI is used to distinguish pathologic tissue such as a tumour in the brain from normal tissue.

Differences in the signal returned by specific tissues in the body produce the image and make it a useful tool for studying anatomy and pathology. The slice images vary from one to ten milimeters thick or more. Images can be collected on the axial (from head to toe), saggital (horizontal), coronal (veins and arteries) and oblique(i.e.: 45 degree angle) planes. Often during an MRI examination, contrast agents are injected into the body to rule out or highlight areas or abnormalities. The procedure of obtaining sufficient data is time consuming, often taking one to three hours. Data is stored and displayed digitally.

The contrast-enhanced magnetic resonance imaging (MRI) signal is rarely a direct measure of contrast concentration; rather it depends on the effect that the contrast agent has on the tissue water magnetization. To correctly interpret such studies, an understanding of the effects of water movement on the magnetic resonance (MR) signal is critical.

Water diffusion within biological compartments and water exchange between biological compartments affects MR signal enhancement and therefore the ability to extract physiologic information. The two primary ways by which contrast agents affect water magnetization are: (1) direct relaxivity and (2) indirect susceptibility effects.

In a gamma ray detection procedure a patient is firstly injected with a gamma-emitting radiopharmaceutical. Then a series of projection images are acquired using a gamma camera. The acquisition involves the gamma camera rotating around the patient acquiring images at various positions. The number of images and the rotation angle covered varies depending on the type of investigation required, but a typical example involves the gamma camera rotating 360 degrees around the patient, acquiring 64 equally spaced images.

Ultrasound, also called diagnostic medical sonography, sonography, and echocardiography, as an imaging method, is harmless and non-invasive to the body. 2D, 3D and 4D (3D in real time) systems are available.

Ultrasound is excellent for non-invasively imaging and diagnosing a number of organs and conditions, without x-ray radiation. Modem obstetric medicine (for guiding pregnancy and child birth) relies heavily on ultrasound to provide detailed images of the fetus and uterus. Ultrasound is also extensively used for evaluating the kidneys, liver, pancreas, heart, and blood vessels of the neck and abdomen. Ultrasound can also be used to guide fine needle, tissue biopsy to facilitate sampling cells from an organ for lab testing (for example, to test for cancerous tissue).

Ultrasound images of flow, whether colour flow or spectral Doppler, are essentially obtained from measurements of movement. In ultrasound scanners, a series of pulses is transmitted to detect movement of blood. Echoes from stationary tissue are the same from pulse to pulse. Echoes from moving scatterers exhibit slight differences in the time for the signal to be returned to the receiver [www.centrus.com]. These differences can be measured as a direct time difference or, more usually, in terms of a phase shift from which the 'Doppler frequency' is obtained. They are then processed to produce either a colour flow display or a Doppler sonogram.

In medical ultrasound, the Doppler frequency shift is the difference between the frequency of the transmitted and reflected ultrasound. This is due a relative movement between the reflector (most frequently the red blood cells) and the ultrasound transducer. The ultrasound apparatus registers this difference in frequency and calculates the linear rate of flow employing the Doppler equation. The Doppler analysis is presented acoustically, graphically or by means of a colour code. In pulsed Doppler technique, conventional ultrasound scanning can be combined with Doppler analysis. Important indications are: differentiation between vascular and non-vascular structure, documentation of flow and determination of the direction of flow, diagnosis and quantitation of arterial stenoses and assessment of transplants.

Based on one or several of the described diagnostic procedures, digital coordinates to regions of interest are established and recorded, based on a well defined coordinate system, linked to reference points on or within the patient. These coordinates are provided to a processing unit, which may include or be provided with accompanying software and/or algorithms, which supports the digital guidance and control of the therapeutic acoustic unit. The processing unit may provide a digital model of the region of interest and subsequently add an automated and/or optimized treatment regime or procedure based on geometry, anatomy and empirical data.

Ultrasound images do not only depend on the local characteristics of the regions of interest, but are also dependent on the intervening medium which the ultrasonic beam has to pass through. To monitor the release of the agent(s) in real time, one would e.g. like to observe changes in the backscattering in the region of interest as the injected drug(s) and/or lipids are exposed to low frequency ultrasound and disintegrate. The fact that only changes are required makes this problem somewhat simpler than the general problem, because changes can be found if the properties of the intervening medium are kept constant. The intervening medium can be assumed to be unchanged if the low frequency ultrasound only targets the region of interest and not the intervening medium. Changes in the region of interest relate to local changes only, and the effect of the low frequency ultrasound can be characterized from intensity changes.

In order to monitor the ultrasonic release of the agent, a diagnostic ultrasonic imager and/or a MRI unit may be used to monitor the tumour before, during and after ultrasonic release (ultrasonic therapeutic exposure).

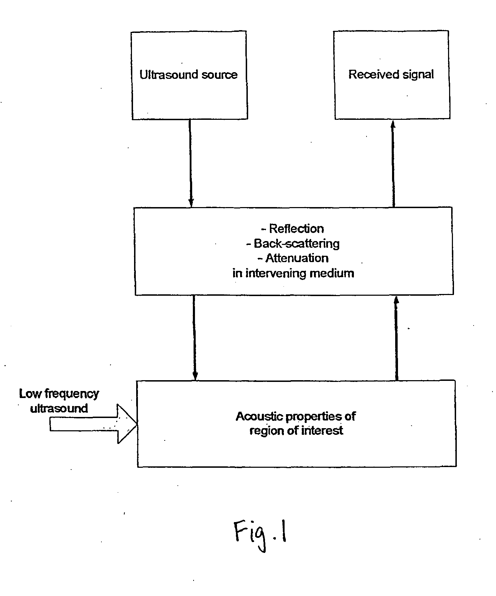

In [Ultrasonics, vol 32, no 2, pp. 123-130, 1994] an acoustic model for the imaging of a region of interest seen through intervening tissue is proposed. The context of the model is interpretation of backscattered signals from ultrasound contrast agents in the cardiac ventricles when viewed through tissue or blood that may also contain a contrast agent. Another independent study of the same phenomenon [Ultrasound in Medicine & Biology, Volume 22, Issue 4, 1996, pp 441-451] confirms this model. Figure 1 shows an adaptation of the model to the problem considered here. As previously stated, cavitational effects in general can be monitored by diagnostic ultrasound units [Biophys J. (2001) 80;1547 - 1556]. The same applies for MRI.

By recording and/or measuring these general cavitational effects, one can calculate the drug release based on empirical data.

There are several factors that influence a beam of ultrasound as it passes through a medium. The first effect is reflection and transmission at each change in acoustic impedance. The acoustic impedance is $Z = \rho c$, the product of the density and the speed of sound. If the changes in acoustic impedance are small, then only a small fraction of the energy is reflected and most of the energy is passed on to larger depths. This is the case for tissue and blood, but not for air and bone, and this explains why it is hard to image through lungs and bones.

The second effect is due to the presence of scatterers in the medium, e.g. a contrast agent. This effect is similar to the previous effect, in that the energy which is not back-scattered will be transmitted to further depths.

The third effect is that tissue has a frequency dependent attenuation coefficient which also varies with the kind of tissue. Thus the observed intensity in a region of interest is a function of the acoustic impedance changes, backscattering, and the attenuation in the intervening tissue as well as the backscattering in the region of interest itself. Ideally one would like to measure and isolate only the backscattering in the region of interest.

In the present application, one would like to observe changes in the backscattering in the region of interest as e.g. the lipids are exposed to the low frequency ultrasound.

The use of harmonic (octave) imaging is not very different from conventional B-mode imaging. The properties of the intervening tissue also influence the level imaged from the region of interest. There could be a slight advantage with non-linear imaging as the ultrasonic source in this case it is not the probe itself, but rather the generation of the second harmonic in the medium. The non-linearity is more pronounced near the intensity maximum, i.e. near or in front of the focal point. Therefore the second harmonic has not passed through all of the intervening tissue on the way to the region of interest. The model for frequency shift in Doppler imaging mode is much simpler and only dependent on the local properties in the region of interest and not the intervening medium. If a Doppler mode can be used for assessing the effect of the low frequency ultrasound exposure, then the problem becomes simple. A mode such as the Doppler power mode needs the above B-mode imaging model for interpretation, only modes involving Doppler velocities depend on local region of interest properties alone.

Several of the diagnostic techniques which have been discussed, can be applied in a thermal monitoring mode, in particular MRI.

In addition, it is concluded that magnetic resonance spectroscopy provides a viable non-invasive means of measuring regional brain temperatures in normal subjects and is a promising approach for measuring temperatures in brain-injured subjects. [J Cereb Blood Flow Metab. 1997 Apr;17(4):363-9].

The human body emits infrared light with the periodicity of the heartbeat. The infrared thermal emission occurs in characteristic patterns which, after the heartbeat, migrate over the body. The images are obtained by using the electrocardiographic signal as a trigger to produce a thermal image which is subtracted from another one taken a well defined time later, before the next heart impulse. When these difference images are frequently repeated and averaged, a heart pulse induced heat turnover is determined which has no relation to conventional heat images of the human body, [Medical Physics, November 2001, Volume 28, Issue 11, pp. 2352-2357].

In Krotov et al [Acoustical monitoring of the internal temperature of biological objects during laser hyperthermia, Proc. XIII Session of the Russian Acoustical Society, Moskow, Aug., 2003] multi channel acoustical thermograph (AT) and acoustical brightness thermometry (ABT) methods for monitoring of the internal temperatures during hyperthermia procedures are discussed.

According to one aspect, the invention provides a method of controlling therapeutic ultrasound treatment by using cavitational data obtained via MRI.

According to another aspect, the invention provides apparatus for non-invasive patient treatment using ultrasound, comprising an ultrasonic transmitting device for focusing ultrasonic energy at a region of interest of a patient, at energy levels sufficient to interact with an encapsulated therapeutic agent, and control means for receiving information about a desired location where the ultrasound energy is to be focused and for controlling the ultrasonic transmitting device so that the ultrasonic energy is focused at that desired location.

According to yet another aspect, the invention provides a method of non-invasive patient treatment using ultrasound, comprising administering to a patient an encapsulated therapeutic agent, transmitting ultrasound and focusing the ultrasonic energy at a region of interest of a patient, at energy levels sufficient to interact with the encapsulated therapeutic agent, and controlling the ultrasonic transmitting device, based on information about a desired location where the ultrasonic energy is to be focused, so that the ultrasonic energy is focused at that desired location.

It is therefore possible to target the release of the therapeutic agent, using the focused ultrasonic energy, at precisely the location where treatment is required.

Certain preferred embodiments of the invention will now be described by way of example only and with reference to the accompanying drawings, in which:

Figure 1 relates to the monitoring, measurement and/or control of the ultrasonic release of the agent;
Figure 2 is a schematic block diagram of a diagnostic and therapeutic system;
Figure 3 is a schematic view of the treatment apparatus; and
Figure 4 is a schematic view showing a diagnostic unit and a treatment apparatus.
Figure 5 is a schematic view showing a treatment apparatus according to an embodiment of the invention.

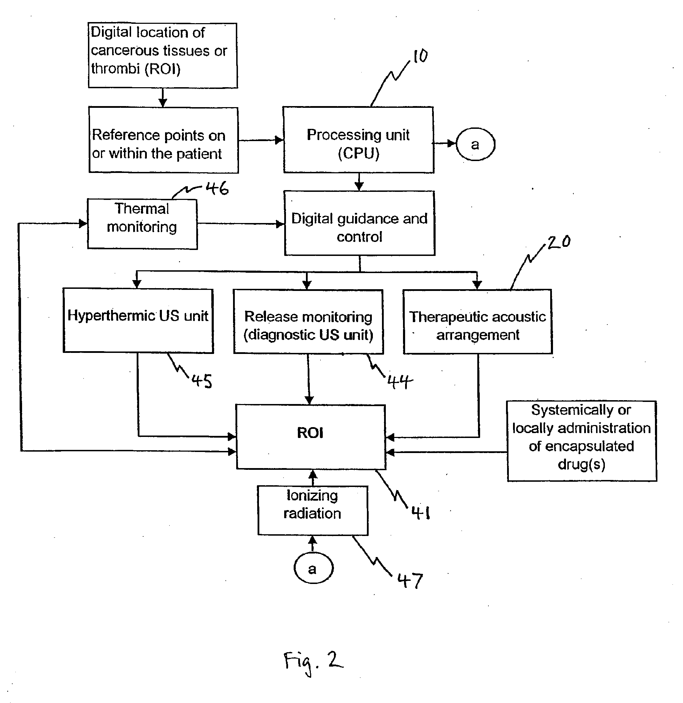

Figures 2, 3 and 4 outline a multi modal patient treatment system for the treatment of cancer or thrombi.

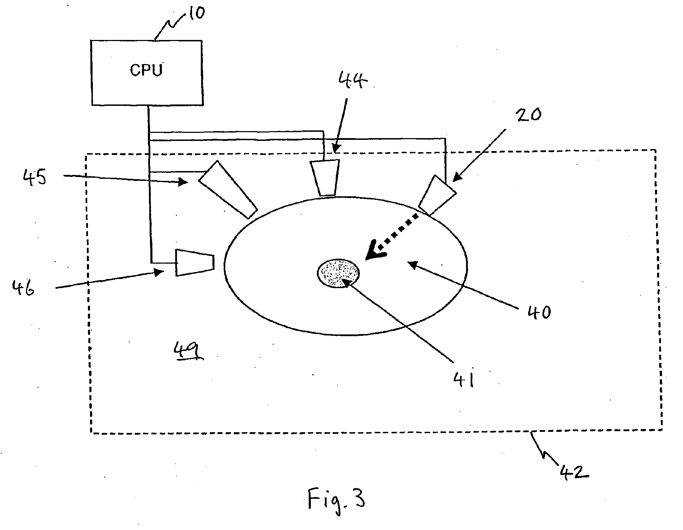

Figure 3 shows a cross-section through a patient 40 having a region of interest and/or a digital model 41 for therapeutic treatment. The patient is immersed, at least partly, in a bath 42 containing water 49 or gel to serve as an ultrasound conducting medium. Various portions of the treatment apparatus are also immersed in the bath 42. These consist of a therapeutic transmitter or transducer 20, a diagnostic transducer 44, a thermal transducer 45 and a thermal monitor 46. Each of these components is connected to a central processing unit 10. One or several of these components can be integrated and/or a component can act in several modes, among; therapy, thermal [destructive (ablation) or hyperthermal], diagnosis, monitoring etc.

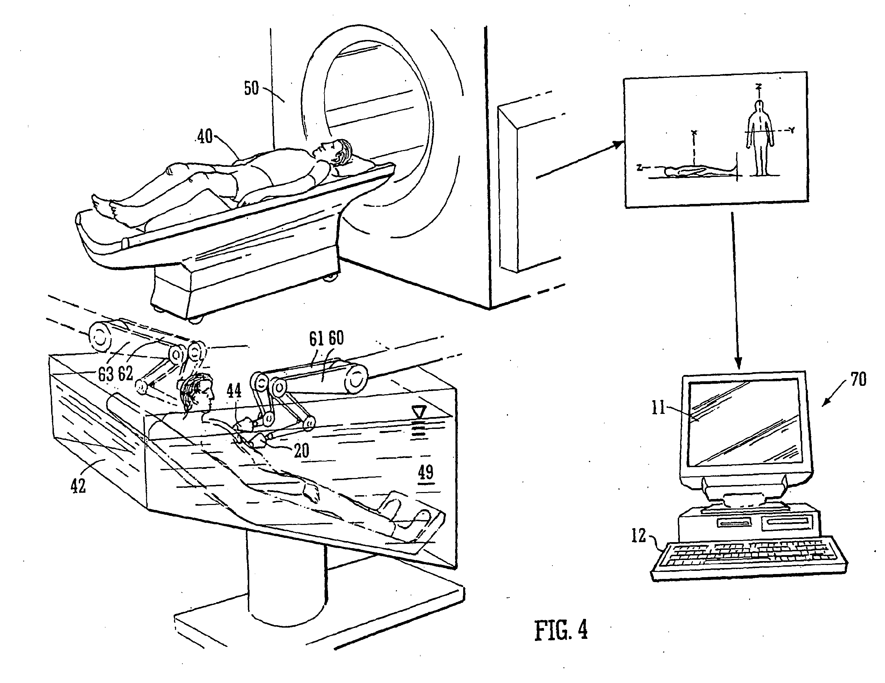

Figure 4 shows a diagnostic scanner 50 for receiving a patient 40 in order to determine the location of a region of interest, such as a cancer or thrombus, which is to be treated. The location is determined and recorded relative to a reference point on the patient (e.g. the tip of the nose or ear) in the "X", "Y" and "Z" directions. These co-ordinates are fed to a control means 70 which includes the central processing unit 10, a screen 11, a keyboard 12 and a mouse (not shown).
Figure 4 also shows the patient at a later stage, immersed up to the neck in a bath 42 of water 49. The therapeutic transmitter 20 is supported at the end of a robotic arm 60 and the diagnostic transducer 44 is supported at the end of a robotic arm 61. Further robotic arms 62 and 63 are shown in dotted lines and serve to support the thermal transducer (hypothermic and/or thermal destructive) 45 and the thermal monitoring device 46. The control means 70 communicates with the robotic arms via respective connecting lines (not shown).

Referring to Figure 1, the system is operated as follows. The starting point is diagnosis and position determination of regions of interest (the location of tumours or thrombi) based on CAT, CT, EBT, PET, SPECT, MRI, ultrasound or combinations thereof, and digital recording of the location of regions of interest with respect to reference points. The coordinates are transferred into a central processing unit (CPU) with appropriate software and/or algorithms 10. The location of the reference points on the patient are recorded into the CPU by a pointer (not shown on the figures) and subsequently the location of the regions of interest is known to the system (CPU). The region of interest (tumour, thrombus, organ or the like) can subsequently be modeled by topographic modeling techniques (morphometry, digital elevation models, tumour profiling etc.). An adequate 3D or 3D + time digital (or analog) tumour model may facilitate the optimal automated or manually controlled treatment regime or procedure based on e.g. combinations of geometry, anatomy and empirical data.

A therapeutic transmitter 20 is then able to be digitally guided and controlled using the data concerning the reference points (or model). This may be automatic and/or optimized, with respect to minimized attenuation, based on a predetermined treatment regime, or the operator may set the point(s) where ultrasound energy is to be focused.

The coordinate information may thus be provided by the diagnostic scanner 50 and used automatically by the control means to direct ultrasound energy to the desired point. Alternatively, the information provided by the scanner 50 may be provided to an operator e.g. on a screen to use his judgement to select where the ultrasound is to be directed, the operator inputting the selection via the keyboard 12 or mouse or other input device and the control means then directing the energy to the desired point by moving the robotic arm 60 or electronically moving the focal point of the ultrasound or a combination of the two.

The therapeutic apparatus may include one or several of the following additional components, which may or may not be integrated and/or at least one component able to act in several modes.

Diagnostic and/or release monitoring unit based on CAT, CT, EBT, PET, SPECT, MRI, ultrasound or combinations thereof.

- Hyperthermic ultrasound unit.
- Thermal tissue destruction ultrasound (ablation) unit.
- Thermal monitoring unit.
- Ionizing radiation unit.

In operating the system, prior or during the treatment, the patient is administered one or combinations of several drugs, encapsulated agents, among cytotoxic, phototherapeutic, radiation sensitizers, anti-angiogenetic agents or cocktails thereof, with or without added micro bubbles or only encapsulated micro bubbles (in addition to naturally occurring bubbles). This may be by a single injection or infusion, or the administration may be made intravenously and controlled in response to information fed back from the monitoring and/or measurements of agent delivery or release caused by the ultrasound.

The therapeutic acoustic arrangement is moved into position by the guidance and control system and the robotic arms.

The drugs and/or encapsulated agents may contain micro bubbles. Microbubbles are also naturally present in fluids and are created by application of ultrasound. Therefore cavitational effects are present and the release and the amount of the active substances can be recorded or calculated in real time by monitoring the cavitation using ultrasound, MRI or the like. This real time monitoring and calculating can then be used to control the therapeutic ultrasound transmitter 20 and/or the application of the therapeutic agent (e.g. drug).

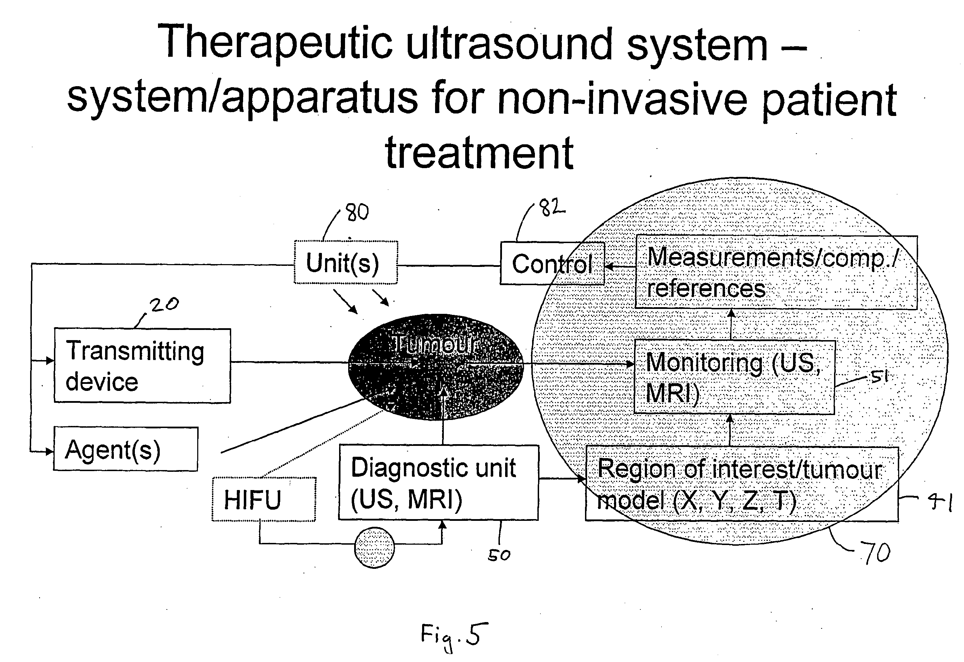

Figure 5 schematically shows an embodiment of the invention. A diagnostic scanner 50 (alternatively this could be the diagnostic unit 44) is used to find the location of the region of interest (e.g. tumour or thrombus), using high frequency ultrasound (HIFU). The diagnostic scanner 50 then communicates the location of the region of interest to the control means 70 where a digital model of the region of interest 41 is created. This model is dependent on spatial co-ordinates X, Y and Z and is also time dependent.

A monitoring means 51 monitors cavitational effects within the region of interest in real time. The monitoring means is either a diagnostic ultrasound unit or a MRI unit. This detected cavitation data is combined (using software and algorithms) with the model of the region of interest 41 and with other measurements and calculations to provide output control signals 82 which are used to control the therapeutic transmitter 20 and to regulate the administration of the drugs/therapeutic agents which are being applied to the region of interest. The control signals can also be used to control other units 80 such as an ionizing radiation unit, a hyperthermal monitoring unit and/or an ablation unit if these are desired as part of the treatment programme.

With this feedback loop, the real time control of the treatment is effected and unwanted damage to tissue surrounding the region of interest is avoided.

The other treatment modes like hyperthermia, thermal ablation or destruction, ionizing radiation may be conducted in parallel or in sequence. Figure 2 shows an option in which the control means 10 provides a signal "a" to an ionizing radiation device 47.

Actual treatment, for all modalities, are conducted according to empirical data, endogenously (provided by or within the system) or exogenously (set by an operator or stopped/interrupted manually).

The system can release any kind of encapsulated substance or enhance the effect of a drug anywhere in the patient.

The present apparatus, system and/or methodology may represent an overall control framework (which may include software and/or algorithms) comprising at least one of the components; a therapeutic ultrasound component, novel and inventive monitoring and control means for ultrasound mediated selective or targeted release of a drug or therapeutic agent, existing technology available for diagnosis (ultrasound, MRI, PET, CAT, CT, CT/X-ray and the like), targeting ultrasound for tissue heating, destruction or ionizing radiation.

The present invention is not limited to the described apparatus and algorithm, thus all devices that are functionally equivalent are included by the scope of the invention.

Drawings and figures are to be interpreted illustratively and not in a limiting context. It is further presupposed that all the claims shall be interpreted to cover all generic and specific characteristics of the invention which are described, and that all aspects related to the invention, no matter the specific use of language, shall be included. Thus, the stated references have to be interpreted to be included as part of this invention's basis, methodology mode of operation and apparatus.

**Claims**

1.  Apparatus for non-invasive patient treatment using ultrasound and a therapeutic agent, comprising an ultrasonic transmitting device (20) for directing ultrasonic energy at a region of interest (41) of a patient (40) and inducing cavitation of naturally occurring microbubbles to increase the interaction between the applied ultrasound and the therapeutic agent, and monitoring means adapted to monitor the cavitation in real time in and around the region of interest, the apparatus further comprising control means (10) for receiving information from the monitoring means and **characterised in that** the control means is for controlling the ultrasonic transmitting device based on that information so that the ultrasonic energy is focused at the region of interest (41) in order to minimise damage to tissue outside the region of interest (41), and **in that** the monitoring means is a magnetic resonance imaging machine adapted to monitor cavitation of naturally occurring microbubbles.

2. Apparatus as claimed in claim 1, in combination with a diagnostic unit (50) for determining the region of interest.

3. Apparatus as claimed in claim 2, wherein the diagnostic unit (50) is provided at a first station and the ultrasonic transmitting device (20) is provided at a second station.

4. Apparatus as claimed in claim 2, wherein the diagnostic unit (50) is also the monitoring means.

5. Apparatus as claimed in claim 2, 3 or 4, wherein the diagnostic unit (50) is arranged to determine the region of interest with respect to a reference point.

6. Apparatus as claimed in claim 5, wherein the reference point is on the patient (40).

7. Apparatus as claimed in any preceding claim, comprising a vessel containing an ultrasound conducting medium and in which the ultrasonic transmitting device (20) is disposed, the vessel being arranged to permit immersion in the ultrasound conducting medium of at least part of a patient's (40) anatomy.

8. Apparatus as claimed in any preceding claim, comprising a support for the ultrasonic transmitting device (20) which is movable to adjust the position of the device relative to the patient (40).

9. Apparatus as claimed in any preceding claim, wherein the monitoring means is arranged to provide quantitative information concerning the effect of the therapeutic agent as a result of the interaction of the therapeutic agent with the ultrasonic energy.

10. Apparatus as claimed in any preceding claim, wherein the therapeutic agent is a drug and the information from the monitoring means is used to calculate the amount of drug uptake within the region of interest (41).

11. Apparatus as claimed in any preceding claim, wherein the monitoring means is arranged to provide information relating to the location where the therapeutic agent is active.

12. Apparatus as claimed in claim 11, wherein the information from the monitoring means is used to spatially model the drug uptake within the region of interest (41).

13. Apparatus as claimed in any preceding claim, comprising a thermal detection device (45) for real time thermal monitoring.

14. Apparatus as claimed in any preceding claim, comprising a sensor for checking the position of the region of interest (41) during treatment.

15. Apparatus as claimed in any preceding claim, wherein the ultrasonic transmitting device (20) is arranged to transmit ultrasound at a frequency less than 1 MHz.

16. Apparatus as claimed in any of claims 1 to 14, wherein the ultrasonic transmitting device (20) is arranged to transmit ultrasound at a frequency between 20 kHz and 250 kHz.

17. Apparatus as claimed in any preceding claim, wherein the apparatus is arranged to deliver ultrasonic energy to the patient in non-hyperthermic conditions.

**Patentansprüche**

1. Vorrichtung für eine nichtinvasive Patientenbehand-lung unter Verwendung von Ultraschall und eines therapeutischen Wirkstoffs, mit einer Ultraschallsendeeinrichtung (20) zum Richten von Ultraschallenergie in einen interessierenden Bereich (41) eines Patienten (40) und zum Induzieren einer Kavitation aus natürlich auftretenden Mikroblasen, um die Wechselwirkung zwischen dem angewendeten Ultraschall und dem therapeutischen Wirkstoff zu erhöhen, und mit Überwachungsmitteln, die dazu ausgelegt sind, die Kavitation in Echtzeit in und um den interessierenden Bereich zu überwachen, wobei die Vorrichtung ferner Steuermittel (10) enthält, um von den Überwachungsmitteln Informationen zu empfangen, **dadurch gekennzeichnet, dass** die Steuermittel dazu dienen, die Ultraschallsendeeinrichtung anhand dieser Informationen zu steuern, so dass die Ultraschallenergie auf den interessierenden Bereich (41) konzentriert wird, um die Beschädigung von Gewebe außerhalb des interessierenden Bereichs (41) minimal zu machen, und dass die Überwachungsmittel eine Magnetresonanz-Abbildungsmaschine bzw. ein Magnetresonanztomograph ist, die bzw. der dazu ausgelegt ist, die Kavitation von natürlich auftretenden Mikroblasen zu überwachen.

2. Vorrichtung nach Anspruch 1 in Kombination mit einer Diagnoseeinheit (50), um den interessierenden Bereich zu bestimmen.

3. Vorrichtung nach Anspruch 2, wobei die Diagnoseeinheit (50) an einer ersten Station vorgesehen ist und die Ultraschallsendeeinrichtung (20) an einer zweiten Station vorgesehen ist.

4. Vorrichtung nach Anspruch 2, wobei die Diagnoseeinheit (50) auch die Überwachungsmittel enthält.

5. Vorrichtung nach Anspruch 2, 3 oder 4, wobei die Diagnoseeinheit (50) dazu ausgelegt ist, den interessierenden Bereich in Bezug auf einen Referenzpunkt zu bestimmen.

6. Vorrichtung nach Anspruch 5, wobei sich der Referenzpunkt an dem Patienten (40) befindet.

7. Vorrichtung nach einem vorhergehenden Anspruch, die ein Gefäß aufweist, das ein Ultraschall leitendes Medium enthält und in dem die Ultraschallsendeeinrichtung (20) angeordnet ist, wobei das Gefäß dazu ausgelegt ist, wenigstens einen Teil der Anatomie des Patienten (40) in das Ultraschall leitende Medium einzutauchen.

8. Vorrichtung nach einem vorhergehenden Anspruch, das einen Träger für die Ultraschallsendeeinrichtung (20), der beweglich ist, um die Position der Einrichtung relativ zu dem Patienten (40) einzustellen, enthält.

9. Vorrichtung nach einem vorhergehenden Anspruch, wobei die Überwachungsmittel dazu ausgelegt sind, quantitative Informationen bezüglich der Wirkung des therapeutischen Wirkstoffs als ein Ergebnis der Wechselwirkung des therapeutischen Wirkstoffs mit der Ultraschallenergie bereitzustellen.

10. Vorrichtung nach einem vorhergehenden Anspruch, wobei der therapeutische Wirkstoff ein Arzneimittel ist und die Informationen von den Überwachungsmitteln verwendet werden, um die in einem interessierenden Bereich (41) aufgenommene Arzneimittelmenge zu berechnen.

11. Vorrichtung nach einem vorhergehenden Anspruch, wobei die Überwachungsmittel dazu ausgelegt sind, Informationen bezüglich der Stelle, an der der therapeutische Wirkstoff aktiv ist, bereitzustellen.

12. Vorrichtung nach Anspruch 11, wobei die Informationen von den Überwachungsmitteln verwendet werden, um die Arzneimittelaufnahme in dem interessierenden Bereich (41) räumlich zu modellieren.

13. Vorrichtung nach einem vorhergehenden Anspruch, die eine Wärmedetektionsvorrichtung (45) für die Wärmeüberwachung in Echtzeit enthält.

14. Vorrichtung nach einem vorhergehenden Anspruch, die einen Sensor zum Prüfen der Position des interessierenden Bereichs (41) während der Behandlung enthält.

15. Vorrichtung nach einem vorhergehenden Anspruch, wobei die Ultraschallsendevorrichtung (20) dazu ausgelegt ist, Ultraschall bei einer Frequenz von weniger als 1 MHz zu senden.

16. Vorrichtung nach einem der Ansprüche 1 bis 14, wobei die Ultraschallsendeeinrichtung (20) dazu ausgelegt ist, Ultraschall bei einer Frequenz im Bereich von 20 kHz bis 250 kHz zu senden.

**17.** Vorrichtung nach einem vorhergehenden Anspruch, wobei die Vorrichtung dazu ausgelegt ist, Ultraschallenergie an den Patienten unter nicht hyperthermischen Bedingungen abzugeben.

**Revendications**

**1.** Dispositif pour un traitement de patient non invasif utilisant des ultrasons et un agent thérapeutique, comportant un dispositif de transmission d'ultrasons (20) pour diriger une énergie ultrasonore dans une région d'intérêt (41) d'un patient (40) et provoquer une cavitation de micro-bulles survenant naturellement afin d'augmenter l'interaction entre les ultrasons appliqués et l'agent thérapeutique, et des moyens de surveillance adaptés pour surveiller la cavitation en temps réel dans la région d'intérêt et autour de celle-ci, le dispositif comportant en outre des moyens de commande (10) pour recevoir des informations en provenance des moyens de surveillance et **caractérisé en ce que** les moyens de commande sont destinés à commander le dispositif de transmission d'ultrasons sur la base de ces informations de sorte que l'énergie ultrasonore est concentrée dans la région d'intérêt (41) afin de minimiser les dommages au tissu à l'extérieur de la région d'intérêt (41) et **en ce que** les moyens de surveillance sont une machine d'imagerie par résonance magnétique adaptée pour surveiller la cavitation de micro-bulles survenant naturellement.

**2.** Dispositif selon la revendication 1, en combinaison avec une unité de diagnostic (50) pour déterminer la région d'intérêt.

**3.** Dispositif selon la revendication 2, dans lequel l'unité de diagnostic (50) est agencée dans une première station et le dispositif de transmission d'ultrasons (20) est agencé dans une seconde station.

**4.** Dispositif selon la revendication 2, dans lequel l'unité de diagnostic (50) constitue également les moyens de surveillance.

**5.** Dispositif selon la revendication 2, 3 ou 4, dans lequel l'unité de diagnostic (50) est conçue pour déterminer la région d'intérêt par rapport à un point de référence.

**6.** Dispositif selon la revendication 5, dans lequel le point de référence est sur le patient (40).

**7.** Dispositif selon l'une quelconque des revendications précédentes, comportant une cuve contenant un support conducteur d'ultrasons et dans lequel le dispositif d'émission d'ultrasons (20) est disposé, la cuve étant conçue pour permettre l'immersion dans le support conducteur d'ultrasons d'au moins une partie de l'anatomie d'un patient (40) .

**8.** Dispositif selon l'une quelconque des revendications précédentes, comportant un support pour le dispositif de transmission d'ultrasons (20) lequel est mobile pour ajuster la position du dispositif par rapport au patient (40).

**9.** Dispositif selon l'une quelconque des revendications précédentes, dans lequel les moyens de surveillance sont conçus pour fournir des informations quantitatives concernant l'effet de l'agent thérapeutique en résultat de l'interaction de l'agent thérapeutique avec l'énergie ultrasonore.

**10.** Dispositif selon l'une quelconque des revendications précédentes, dans lequel l'agent thérapeutique est un médicament et les informations en provenance des moyens de surveillance sont utilisées pour calculer la quantité d'absorption de médicaments dans la région d'intérêt (41).

**11.** Dispositif selon l'une quelconque des revendications précédentes, dans lequel les moyens de surveillance sont conçus pour délivrer des informations concernant l'emplacement où l'agent thérapeutique est actif.

**12.** Dispositif selon la revendication 11, dans lequel les informations en provenance des moyens de surveillance sont utilisées pour modéliser spatialement l'absorption de médicaments dans la région d'intérêt (41).

**13.** Dispositif selon l'une quelconque des revendications précédentes, comportant un dispositif de détection thermique (45) pour une surveillance thermique en temps réel.

**14.** Dispositif selon l'une quelconque des revendications précédentes, comportant un détecteur pour contrôler la position de la région d'intérêt (41) pendant un traitement.

**15.** Dispositif selon l'une quelconque des revendications précédentes, dans lequel le dispositif de transmission d'ultrasons (20) est conçu pour transmettre des ultrasons à une fréquence inférieure à 1MHz.

**16.** Dispositif selon l'une quelconque des revendications 1 à 14, dans lequel le dispositif de transmission d'ultrasons (20) est conçu pour transmettre des ultrasons à une fréquence comprise entre 20 kHz et 250 kHz.

**17.** Dispositif selon l'une quelconque des revendications précédentes, dans lequel le dispositif est conçu pour délivrer de l'énergie ultrasonore au patient dans des conditions non hyperthermiques.

| Ultrasound source | | Received signal |

| - Reflection<br>- Back-scattering<br>- Attenuation<br>in intervening medium |

**Low frequency<br>ultrasound** ⟹ | Acoustic properties of<br>region of interest |

Fig. 1

Digital location of cancerous tissues or thrombi (ROI)

Reference points on or within the patient

Processing unit (CPU) — *10*

a

Thermal monitoring — *46*

Digital guidance and control

Hyperthermic US unit — *45*

Release monitoring (diagnostic US unit) — *44*

Therapeutic acoustic arrangement — *20*

ROI

Systemically or locally administration of encapsulated drug(s)

Ionizing radiation — *41*

a — *47*

Fig. 2

Fig. 3

FIG. 4

# Therapeutic ultrasound system – system/apparatus for non-invasive patient treatment

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0215976 A
- WO 05002671 A
- US 5753259 A
- US 6656385 B
- US 5275165 A
- US 5443068 A
- US 5769790 A
- US 6461586 B1
- WO 2004075987 A

### Non-patent literature cited in the description

- *Farmacological Rev.,* 1999, vol. 51 (4), 692-737
- *Clin. Cancer Res.,* 2001, vol. 7, 3314-3324
- *Cancer,* 2002, vol. 94 (1), 25-36
- Annals of Oncology. 2004, vol. 15, 440-4493
- *Cancer Chemother. Pharmacol.,* 2004, vol. 53 (5), 452-457
- *Breast,* 2004, vol. 13 (3), 219-2266
- *Br. J. Cancer,* 2002, vol. 87 (6), 600-6007
- *Br. J. Cancer,* 2004, vol. 9 (12), 2402-24107
- *Cancer Res,* 2002, vol. 62 (24), 7280-7283
- *Cancer Letters,* 2006, vol. 232, 206-213
- *Ultrastruct Pathol.,* September 2004, vol. 28 (5-6), 333-40
- *Oncology,* December 2003, vol. 17 (12), 23-8
- *Journal of Nuclear Medicine,* vol. 46 (1), 187S-190S
- *Current Cancer Drug Targets,* November 2004, vol. 4 (7), 555-567
- *Clin Cancer Res.,* January 2004, vol. 10 (2), 415-27
- *Int J Hyperthermia,* November 2004, vol. 20 (7), 781-802
- **Brennen, C.S.** Cavitation and bubble dynamics. Oxford University Press, 1995
- **Ultrasound.** *Med. & Biol.,* 1991, vol. 17, 179-185
- *Nature Reviews,* 2005, vol. 80, 255-260
- *Investigative Radiology,* vol. 33 (12), 886-892
- *European Journal of Radiology,* 2002, vol. 42, 160-168
- **Nelson.** *Cancer Res,* 2002, vol. 62 (24), 7280-7283
- *Phys. Med. Biol.,* 2003, vol. 48, 167-182
- *Phys. Med. Biol.,* 2005, vol. 50, 1821-1836
- *Ultrasonics,* 1994, vol. 32 (2), 123-130
- *Ultrasound in Medicine & Biology,* 1996, vol. 22 (4), 441-451
- *Biophys J.,* 2001, vol. 80, 1547-1556
- *J Cereb Blood Flow Metab.,* April 1997, vol. 17 (4), 363-9
- *Medical Physics,* November 2001, vol. 28 (11), 2352-2357
- **Krotov et al.** Acoustical monitoring of the internal temperature of biological objects during laser hyperthermia. *Proc. XIII Session of the Russian Acoustical Society,* October 2003